(19) Europäisches Patentamt
European Patent Office
Office européen des brevets

(11) Veröffentlichungsnummer: **0 032 684**
**B1**

# (12) EUROPÄISCHE PATENTSCHRIFT

(45) Veröffentlichungstag der Patentschrift: **21.11.85**

(51) Int. Cl.[4]: **C 07 D457/12**

(21) Anmeldenummer: **81100110.6**

(22) Anmeldetag: **09.01.81**

(54) **Verfahren zur Herstellung von 8-alpha-substituierten 6-Methylergolinen.**

(30) Priorität: **16.01.80 DE 3001752**

(43) Veröffentlichungstag der Anmeldung: **29.07.81 Patentblatt 81/30**

(45) Bekanntmachung des Hinweises auf die Patenterteilung: **21.11.85 Patentblatt 85/47**

(84) Benannte Vertragsstaaten: **AT BE CH DE FR GB IT LI LU NL SE**

(56) Entgegenhaltungen:
**CH-A- 386 439**
**DE-A- 2 124 640**
**DE-A- 2 238 540**
**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder: **Sauer, Gerhard, Dr.**
**Königsbacher Zeile 41a**
**D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 032 684 B1

Jouve, 18, rue St-Denis, 75001 Paris, France

**Beschreibung**

Die Erfindung betrifft ein Verfahren zur Herstellung von 8α-substituierten 6α-Methyl-10α-H-ergolinen gemäß dem Anspruch.

Die erfindungsgemäß herstellbaren Verbindungen sind entweder selbst biologisch wirksame Verbindungen oder Zwischenprodukte zur Herstellung derselben.

Es ist bekannt, daß man bei der katalytischen Hydrierung der 9.10-Doppelbindung aus 8β-substituierten Didehydroergolinen glatt die trans-verknüpften 10α-H-Ergoline erhält.

Anders sieht es dagegen bei den 8α-substituierten 9.10-Didehydroergolinen aus. Die katalytische Hydrierung von 8α-substituierten 9.10-Didehydroergolinen führt stets zu Gemischen bestehend aus den trans-verknüpften 10α-H-Ergolinen und den cis-verknüpften 10β-H-Ergolinen. Biologisch wirksame Verbindungen leiten sich jedoch nur von den trans-verknüpften Ergolinen ab, wie z. B. die bekannten Ergoline Dironyl, Sulergin und Delergotril.

So erhält man bei der katalytischen Hydrierung von Lisurid zum Dironyl mit Raney-Nickel in Abhängigkeit vom Katalysator und dem Lösungsmittel maximale Ausbeuten von 66 % an gewünschtem Produkt (DE OS 2 238 540), wobei das Verhältnis von gewünschten 10α-H-Isomeren zu unerwünschten 10β-H-Isomeren etwa 4 : 1 beträgt.

Des weiteren ist aus der DE-A-2 124 640 die Herstellung von Dihydrolyserysäureamid durch Reduktion von Lyserysäureamid mit Alkalimetall (Natrium) in flüssigem Ammoniak bekannt. Dieses Verfahren wird bei einer Temperatur von höchstens − 40 °C und gegebenenfalls unter Zusatz von Alkohol, Äther oder Tetrahydrofuran durchgeführt.

Aufgabe der vorliegenden Erfindung war es, ein Verfahren bereitzustellen, mit welchem 8α-substituierte 9.10-Dihydroergoline sich selektiv zu den entsprechenden 10α-H-Ergolinen reduzieren lassen.

Es wurde nun gefunden, daß sich 8α-substituierte 9.10-Dihydroergoline mit Alkali- oder Erdalkalimetallen in Ammoniak in Gegenwart eines Arylamins bei Temperaturen von − 30 bis − 70 °C in hoher Ausbeute zu den gewünschten 10α-H-Ergolinen reduzieren lassen, ohne daß bei Gemische von 10α-H- und 10β-H-Isomeren auftreten.

Der Verlauf der erfindungsgemäßen Reaktion war insofern überraschend, da der Fachmann in Analogie zu den anderen Hydrierungsverfahren ein Gemisch von 10α-H- und 10β-H-Isomeren erwartet hätte.

Das erfindungsgemäße Verfahren wird zweckmäßigerweise so durchgeführt, daß man das Alkali- oder Erdalkalimetall wie Lithium, Natrium, Kalium oder Calcium in Ammoniak bei Temperaturen unter − 30 °C löst. Anschließend wird das zu hydrierende Ausgangsmaterial und ein Arylamin hinzugegeben, wobei es zweckmäßig ist, einen Lösungsvermittler hinzuzunehmen. Geeignete Lösungsmittel sind beispielsweise Ether wie Diethylether, Methylethylether, Dimethoxyethan, Tetrahydrofuran und Dioxan.

Geeignete Arylamine im Sinne der vorliegenden Erfindung sind Anilin, Methylanilin, Anisidin, o-Dianisidin, Diphenylamin, Ethylanilin, p-Phenylendiamin, Dimethoxyanilin, Phenylhydrazin, Dinitrophenylhydrazin und Benzidin. Das verwendete Arylamin wird in einer Menge von 0,5-5, vorzugsweise 1-2 Moläquivalenten zum Reaktionsgemisch gegeben.

Die Reaktion benötigt zum Ablauf etwa 20-200 min und ist zumeist nach 30-40 min bereits vollständig abgelaufen.

Die nachfolgenden Beispiele sollen das erfindungsgemäße Verfahren erläutern.

Beispiel 1

In etwa 10 ml wasserfreiem Ammoniak werden bei − 70 °C 100 mg Lithium gelöst und bei dieser Temperatur 1 mMol 3-(9.10-Didehydro-6-methyl-8α-ergolinyl)-1.1-diethylharnstoff I und 1,5 mMol Anilin in 5 ml Tetrahydrofuran innerhalb einiger Minuten hinzugefügt. Entfärbt sich die Lösung, wird eine kleine Menge Lithium noch nachgegeben. Dann wird 30 min bei − 70 °C gerührt, mit Ammoniumchlorid bis zur Entfärbung versetzt und der Ammoniak abgedampft. Der Rückstand wird in gesättigter Natriumhydrogencarbonat-Lösung aufgenommen, mit Kochsalz gesättigt und mit Chloroform oder Essigester ausgeschüttelt. Die organische Phase wird mit Natriumsulfat getrocknet, eingedampft und der Rückstand aus Ethanol kristallisiert. Es wird 3-(6-Methyl-8α-ergolinyl)-1.1-diethylharnstoff II in 85 %iger Ausbeute erhalten.

$[\alpha]_D = + 29°$.

Beispiel 2

Analog Beispiel 1 wird aus Isolysergsäureamid III das 6-Methyl-ergolin-8α-carbonsäureamid IV, aus Isolysergsäure-diethylamid V das 6-Methyl-ergolin-8α-carbonsäurediethylamid VI, aus Isolysergsäurehydrazid VII das 6-Methyl-ergolin-8α-carbonsäurehydrazid VIII und aus Isolysergol IX das 6-Methyl-ergolin-8α-methanol X erhalten.

Folgende Versuche wurden ausgeführt :

| Ausgangs-material | Metall | Arylamin | End-produkt | Ausbeute (%) | $[\alpha]_D^o$ |
|---|---|---|---|---|---|
| I * | Li | - | II | 70 | +30 |
| I | Li | Anilin | II | 85 | +29 |
| I | Li | Methylanilin | II | 72 | +29 |
| I | K | Anilin | II | 75 | +29 |
| I | Li | Anisidin | II | 94 | +30 |
| I | Li | o-Dianisidin | II | 90 | +29 |
| I | Li | Diphenylamin | II | 75 | +29 |
| I | Li | p-Phenylen-diamin | II | 71 | +30 |
| III | Li | Anilin | IV | 92 | + 2 |
| III | Na | Anilin | IV | 81 | + 1 |
| V | Li | Anilin | VI | 78 | -67 |
| VII * | Li | - | VIII | 69 | -23 |
| VII | Li | Anilin | VIII | 87 | -24 |
| IX | Li | Anilin | X | 86 | -70 |
| I | Li | 3.5-Dimeth-oxy-anilin | II | 73 | +30 |
| I | Li | Benzidin | II | 77 | +30 |
| I | Li | Phenyl-hydrazin | II | 90 | +30 |
| I | Li | 2.4-Dinitro-phenylhydrazin | II | 71 | +30 |

* Vergleichsversuch.

## Patentansprüche

1. Verfahren zur Herstellung von 8α-substituierten 6-Methyl-10α-H-ergolinen der allgemeinen Formel

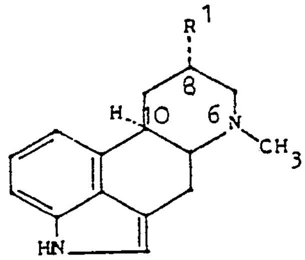

wobei $R^1$ $NH—CO—NR_2$, $CO—NH—NR_2$, $CO—NR_2$ und $CH_2OR$ ist (worin R jeweils Wasserstoff, Methyl

oder Ethyl bedeutet), durch Reduktion von entsprechenden 8α-substituierten 9,10-Didehydro-6-methyl-ergolinen mit Alkalimetall in flüssigem Ammoniak, dadurch gekennzeichnet, daß man die Reduktion in Gegenwart eines Arylamins aus der Gruppe Anilin, Methylanilin, Anisidin, o-Dianisidin, Diphenylamin, Ethylanilin, p-Phenylendiamin, Dimethoxyanilin, Phenylhydrazin, Dinitrophenylhydrazin und Benzidin in einer Menge von 0,5-5 Moläquivalenten bezogen auf das eingesetzte Ergolin ausführt.

2. Verfahren nach Anspruch 1, dadurch gekennzeichnet, daß man als Arylamin Anilin, Anisidin und Phenylhydrazin verwendet.

3. Verfahren nach Anspruch 1, dadurch gekennzeichnet, daß man als Alkalimetall Lithium verwendet.

**Claims**

1. Process for the preparation of 8α-substituted 6-methyl-10α-H-ergolines of the general formula :

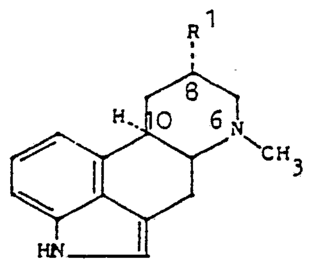

in which $R^1$ is $NH—CO—NR_2$, $CO—NH—NR_2$, $CONR_2$ or $CH_2OR$ (wherein R in each case represents hydrogen, methyl or ethyl) by reduction of corresponding 8α-substituted 9,10-didehydro-6-methylergolines with alkali-metal in liquid ammonia, characterised in that the reduction is carried out in the presence of an arylamine from the group aniline, methylaniline, anisidine, o-dianisidine, diphenylamine, ethylaniline, p-phenylenediamine, dimethoxyaniline, phenylhydrazine, dinitrophenylhydrazine and benzidine in an amount of 0.5-5 molar equivalents relative to the ergoline present.

2. Process according to claim 1, characterised in that aniline, anisidine or phenylhydrazine is used as the arylamine.

3. Process according to claim 1, characterised in that lithium is used as the alkali-metal.

**Revendications**

1. Procédé de préparation de méthyl-6 H-10α ergolines substituées en 8α qui répondent à la formule générale :

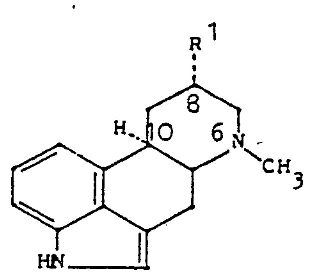

dans laquelle $R^1$ représente un radical $—NH—CO—NR_2$, $—CO—NH—NR_2$, $—CO—NR_2$ ou $—CH_2OR$, où les R représentent chacun l'hydrogène, un méthyle ou un éthyle, par réduction de didéhydro-9,10 méthyl-6 ergolines substituées en 8α correspondantes au moyen d'un métal alcalin dans de l'ammoniac liquide, procédé caractérisé en ce qu'on effectue la réduction en présence d'une arylamine choisie dans l'ensemble constitué par l'aniline, la méthylaniline, l'anisidine, l'o-dianisidine, la diphénylamine, l'éthylaniline, la p-phénylènediamine, la diméthoxyaniline, la phénylhydrazine, la dinitrophénylhydrazine et la benzidine, utilisée en une quantité de 0,5 à 5 équivalents molaires par rapport à l'ergoline mise en jeu.

2. Procédé selon la revendication 1 caractérisé en ce qu'on utilise, comme arylamine, l'aniline, l'anisidine ou la phénylhydrazine.

3. Procédé selon la revendication 1 caractérisé en ce qu'on utilise le lithium comme métal alcalin.